(19)

Europäisches
Patentamt

European
Patent Office

Office européen
des brevets

(11)  **EP 2 881 039 A1**

(12)  **EUROPEAN PATENT APPLICATION**
published in accordance with Art. 153(4) EPC

(43) Date of publication:
**10.06.2015 Bulletin 2015/24**

(21) Application number: **13825580.7**

(22) Date of filing: **30.07.2013**

(51) Int Cl.:
**A61B 6/03** (2006.01)

(86) International application number:
**PCT/JP2013/070659**

(87) International publication number:
**WO 2014/021349 (06.02.2014 Gazette 2014/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **30.07.2012 US 201213561674**

(71) Applicants:
• **Kabushiki Kaisha Toshiba
Minato-ku
Tokyo 105-8001 (JP)**

• **Toshiba Medical Systems Corporation
Otawara-shi, Tochigi 324-8550 (JP)**

(72) Inventors:
• **ZENG, Gengsheng Lawrence
Vernon Hills, Illinois 60061 (US)**
• **ZAMYATIN, Alexander
Vernon Hills, Illinois 60061 (US)**

(74) Representative: **Granleese, Rhian Jane
Marks & Clerk LLP
90 Long Acre
London WC2E 9RA (GB)**

(54)  **X-RAY COMPUTER TOMOGRAPHY IMAGE PICK-UP DEVICE AND IMAGE RECONSTRUCTION METHOD**

(57)  An FBP algorithm is used to substantially reduce noise in an image without losing computational efficiency. A noise weight determining unit (114A) determines a weight value for performing weighting on projection data according to at least a predetermined noise model. A filter shaping unit (114B) shapes at least one reconstruction filter based on parameters including the weight value. An image generation unit (114C) generates an image based on the reconstruction filter.

Noise weight determining unit ~ 114A ~ 114

Filter shaping unit ~ 114B

Image generation unit ~ 114C

FIG. 2

EP 2 881 039 A1

**Description**

**Technical Field**

Embodiments described herein relate generally to an X-ray computed tomography apparatus and an image reconstruction method.

**Background Art**

The filtered backprojection (FBP) algorithm is simple and efficient and is used to reconstruct images in nuclear medicine, X-ray CT, and MRI. The FBP algorithm has been the workhorse in X-ray CT and nuclear medicine image reconstruction thanks to its computational efficiency. If FBP is applied to data acquired at a low dose of X-rays, it undesirably generates noisy images.

As a general trend, the FBP algorithm is gradually being replaced by iterative algorithms. The FBP algorithm undesirably generates images with noise. Furthermore, it is known that the FBP algorithm is not capable of incorporating a noise model for reducing the noise level. In this regard, iterative algorithms can incorporate a projection noise model and generate less noisy images than the FBP algorithm. However, iterative algorithms require intense computation.

Despite the computational requirements, iterative algorithms can advantageously generate noise-resolution balanced images using maximum a posteriori (MAP). To the contrary, it is known that the FBP algorithm is not capable of taking advantage of MAP or prior image information.

**Summary of Invention**

**Technical Problems**

The present invention has as its object to provide an X-ray computed tomography apparatus and an image reconstruction method which can substantially reduce noise in an image using the FBP algorithm without losing computational efficiency.

**Solution to Problems**

According to the present invention, there is provided an X-ray computed tomography apparatus comprising: a determining unit configured to determine a weight value for performing weighting on projection data according to at least a predetermined noise model; a shaping unit configured to shape at least one reconstruction filter based on parameters including the weight value; and a generation unit configured to generate an image based on the reconstruction filter.

**Advantageous Effects of Invention**

It is possible to substantially reduce noise in an image using the FPB algorithm without losing computational efficiency.

**Brief Description of Drawings**

[FIG. 1] FIG. 1 is a view showing the arrangement of an X-ray computed tomography apparatus according to an embodiment.
[FIG. 2] FIG. 2 is a block diagram showing the arrangement of a reconstruction device shown in FIG. 1.
[FIG. 3] FIG. 3 is a flowchart illustrating a typical processing procedure executed by the reconstruction device shown in FIG. 2.
[FIG. 4] FIG. 4 is a view showing a scheme of determining a noise weight value for each view in an image reconstruction method according to the embodiment.
[FIG. 5] FIG. 5 is a view showing a scheme of determining a noise weight value for each ray in the image reconstruction method according to the embodiment.
[FIG. 6A] FIG. 6A is a flowchart illustrating the typical procedure of an image reconstruction method including an additional process of generating an image based on a reconstruction filter shaped in advance according to the embodiment.
[FIG. 6B] FIG. 6B is a flowchart illustrating the typical procedure of another image reconstruction method including an addition process of generating an image based on a reconstruction filter shaped in advance according to the embodiment.

[FIG. 7] FIG. 7 is a view showing the typical procedure of an image reconstruction method using a noise weighted ramp filter in the FBP algorithm.

[FIG. 8] FIG. 8 is a view showing the typical procedure of an image reconstruction method using a pair of noise weighted ramp filters in an Edge-preserving FBP-MAP algorithm.

[FIG. 9] FIG. 9 is a view showing the typical procedure of edge map generation processing shown in FIG. 8.

[FIG. 10] FIG. 10 is a view showing the typical procedure of an image reconstruction method based on an FBP-MAP algorithm using a prior image according to the embodiment.

[FIG. 11] FIG. 11 is a flowchart illustrating the typical procedure of an image reconstruction method using a noise-weighted iteration-emulation FBP algorithm according to the embodiment.

[FIG. 12] FIG. 12 is a view conceptually showing the effect of emulated iteration in the noise weighted FPB algorithm according to the embodiment.

[FIG. 13A] FIG. 13A is a view showing a transverse slice image of the torso region of a cadaver, which is reconstructed based on a conventional FBP Feldkamp algorithm.

[FIG. 13B] FIG. 13B is a view showing a transverse slice image of the torso region of the cadaver, which is reconstructed based on rFBP-reconstruction as rFBP-MAP reconstruction with $\beta = 0$.

[FIG. 13C] FIG. 13C is a view showing a transverse slice image of the torso region of the cadaver, which is reconstructed based on rFBP-MAP reconstruction with $\beta = 0.0005$ using a quadratic smoothing prior.

[FIG. 13D] FIG. 13D is a view showing a transverse slice image of the torso region of the cadaver, which is reconstructed based on rFBP-MAP reconstruction using an edge-preserving prior that is essentially the weighted addition of FIGS. 13B and 13C.

[FIG. 13E] FIG. 13E is a view showing an edge map used for edge-preserving FBP-MAP reconstruction.

[FIG. 13F] FIG. 13F is a view showing a transverse slice image of the torso region of the cadaver, which is reconstructed based on vFBP-MAP reconstruction.

**Description of Embodiment**

An embodiment relates to an X-ray computed tomography apparatus for processing projection data using a noise-weighted filtered backprojection method, and an image reconstruction method.

An X-ray computed tomography apparatus and an image reconstruction method according to the embodiment will be described below with reference to the accompanying drawings.

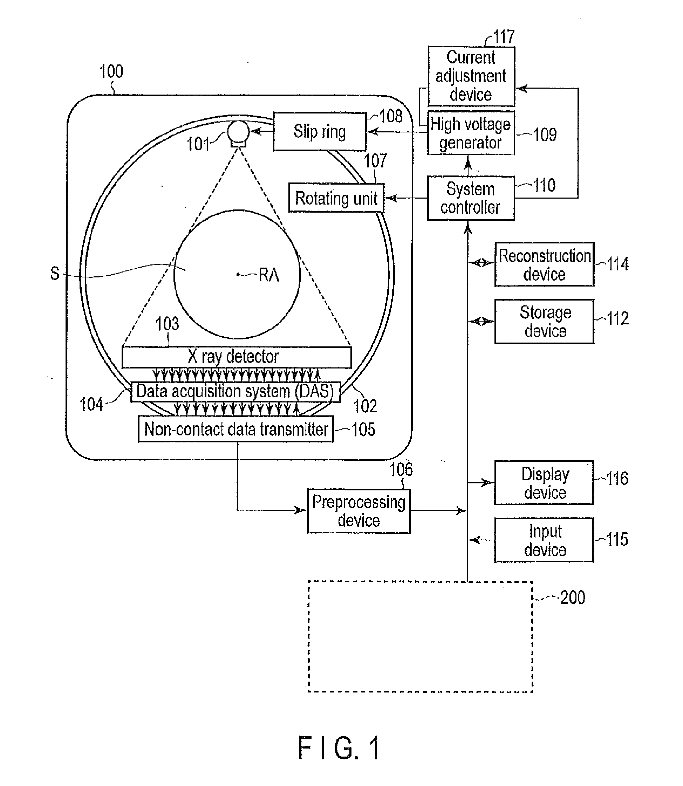

FIG. 1 is a view showing the arrangement of an X-ray computed tomography apparatus according to the embodiment. As shown in FIG. 1, the X-ray computed tomography apparatus according to the embodiment is a multi-slice X-ray CT apparatus or scanner. The X-ray computed tomography apparatus according to the embodiment includes a gantry 100. The gantry 100 includes an X-ray tube 101, an annular frame 102, and a multi-row type or two-dimensional array type X-ray detector 103. The X-ray tube 101 and the X-ray detector 103 are diametrically mounted across a subject S on the frame 102 which rotates around an axis RA. A rotating unit 107 rotates the frame 102 at a high speed such as 0.4 sec/rotation while the subject S is moved along the axis RA in a direction going into or coming out of the drawing page.

The X-ray computed tomography apparatus according to the embodiment includes a high voltage generator 109. The high voltage generator 109 applies a tube voltage to the X-ray tube 101 so that the X-ray tube 101 generates X-rays. For example, the high voltage generator 109 is mounted on the frame 102. The X-rays are emitted towards the subject S. The X-ray detector 103 is located on the opposite side of the X-ray tube 101 with respect to the subject S for detecting the X-rays having been transmitted through the subject S.

As shown in FIG. 1, the X-ray computed tomography apparatus according to the embodiment further includes other devices for processing signals detected by the X-ray detector 103. A Data Acquisition System (DAS) 104 converts a signal output from the X-ray detector 103 into a voltage signal for each channel, amplifies the voltage signal, and converts the amplified voltage signal into a digital signal. The X-ray detector 103 and the DAS 104 are configured to process a predetermined total number of projections per rotation (TPRP).

The above-described data is transmitted to a preprocessing device 106 which is housed in a console outside the gantry 100 via a non-contact data transmitter 105. The preprocessing device 106 performs preprocessing such as sensitivity correction on raw data. The data having undergone preprocessing is called projection data. A storage device 112 stores the projection data. The storage device 112 is connected to a system controller 110 through a data/control bus, together with a reconstruction device 114, a display device 116, an input device 115, and a scan plan support apparatus 200. The scan plan support apparatus 200 includes a function for supporting an imaging technique to develop a scan plan.

According to the embodiment, the reconstruction device 114 reconstructs an image from the projection data stored in the storage device 112 based on a filtered backprojection (FBP) technique with noise weighting. Alternatively, the reconstruction device 114 may reconstruct an image from the projection data based on a filtered backprojection (FBP) technique with noise weighting and prior in such as a reference image. One of the above-described two examples of the reconstruction device 114 reconstructs an image from the projection data based on a filtered backprojection technique with an additional feature of emulating a specific iteration result in a predetermined number of iterations according to a predetermined iterative reconstruction algorithm.

The reconstruction device 114 is implemented by a combination of software and hardware and is not limited to a particular implementation. The reconstruction device 114 according to the embodiment is applicable to other modalities such as nuclear medicine and magnetic resonance imaging (MRI).

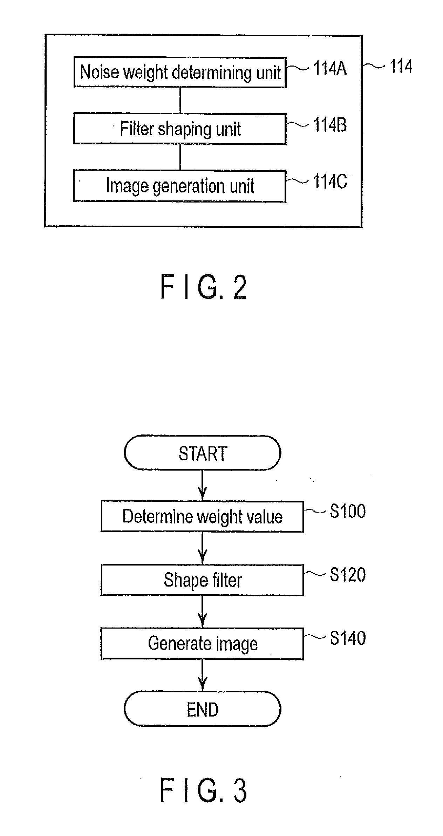

FIG. 2 is a view showing the arrangement of the reconstruction device 114 according to the embodiment. As shown in FIG. 2, the reconstruction device 114 includes a noise weight determining unit 114A, a filter shaping unit 114B, and an image generation unit 114C. The noise weight determining unit 114A determines a weight value for performing weighting on the projection data according to at least a predetermined noise model. The filter shaping unit 114B shapes at least one reconstruction filter based on parameters including the determined weight value. The image generation unit 114C generates an image based on the shaped reconstruction filter. Note that the implementation of the reconstruction device 114 is merely an example. The noise weight determining unit 114A, the filter shaping unit 114B, and the image generation unit 11C will be described in detail below.

FIG. 3 is a flowchart illustrating a typical processing procedure executed by the reconstruction device 114. In step S100, the noise weight determining unit 114A determines a weight value for performing weighting on the projection data according to at least a predetermined noise model. A weight value is determined for each predetermined unit of the projection data. The data unit may be a view or ray. In step S120, the filter shaping unit 114B shapes at least one reconstruction filter based on parameters including the weight value determined in step S100. The parameters are not limited to the weight value while a plurality of reconstruction filters are optionally used for shaping according to the embodiment. In step S140, the image generation unit 114C generates an image based on the reconstruction filter shaped in step S120.

In step S120, a filter is shaped based on predetermined parameters. As described above, one exemplary parameter is the variance of noise components (to be referred to as a noise variance hereinafter) contained in the projection data. The parameters including the weight value according to the embodiment are not limited to a noise parameter. For example, the reconstruction filter may be shaped based on a system matrix. More specifically, the system matrix designates nonuniform sampling of the projection data. Although the conventional FBP algorithm assumes that an object is uniformly sampled, it is also possible to use variable sampling strategies. For example, a signal falling within a given angle range including an important structure is sampled at a first angle interval. To the contrary, a signal falling within another given angle range outside the region of interest is sampled at a second angle interval larger than the first angle interval.

Note that step S100 need not be executed or completed before step S120 in determining a weight value for each predetermined data portion of the projection data. That is, a weight value may be optionally determined every time a reconstruction filter is shaped.

As shown in FIG. 3, in step S140, the image generation unit 114C executes a predetermined set of sub steps while applying the reconstruction filter shaped in step S120. In one exemplary process, in a predetermined FBP technique, after a Fourier transform, an image is reconstructed by applying the shaped filter to the projection data in the frequency domain, as will be described in more detail. In another exemplary process, in a predetermined FBP technique, an image is reconstructed by applying a customized filter to the projection data in the spatial domain as convolution.

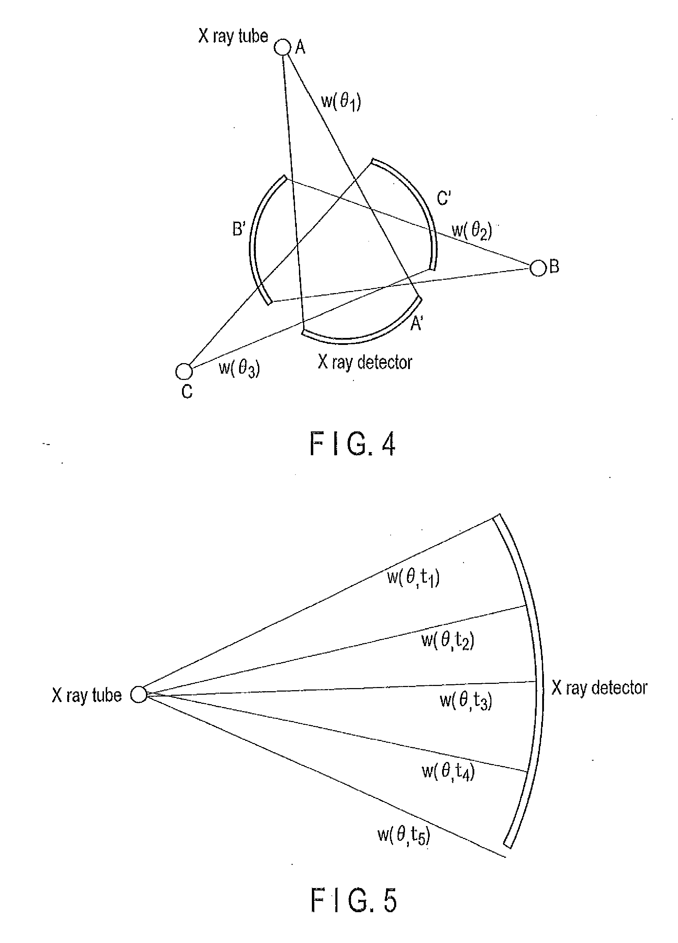

FIG. 4 is a view showing a scheme of determining a noise weight value for each view in an image reconstruction method according to the embodiment. In the view-by-view weighting scheme, each view is generally assigned with a weight value according to a predetermined function such as a maximum or average noise variance in a specific view. FIG. 4 illustrates an X-ray source at three positions A, B, and C and an X-ray detector at three corresponding positions A', B', and C'. If, for example, the X-ray source is at the position A, the X-ray detector is at the corresponding position A'. Projection data acquired by the X-ray detector at the position A' consists of views, and a weight value $w(\theta_1)$ is determined as a function of a view angle $\theta_1$ according to the predetermined view-by-view weighting scheme. As described above, in one exemplary process, determination of a weight value for each view is performed and completed before shaping a reconstruction filter. In another exemplary process, a weight value is determined for each view every time a reconstruction filter is shaped.

In this embodiment, the noise variance contained in the projection data can be modeled using the view-by-view weighting scheme. In general, a typical approach of reconstructing an image while compensating for the influence of noise is to minimize a weighted objective function according to the noise variance, as given by:

$$\|P - AX\|_W^2 \qquad (1)$$

where A represents a projection matrix, X represents an image array described as a column vector, P represents a projection array described as a column vector, and W represents a noise weighting matrix which defines a weighted norm. The objective function is minimized using an iterative Landweber algorithm defined by:

$$X^{(k+1)} = X^{(k)} + \alpha A^T W (P - AX^{(k)}) \qquad (2)$$

where AT represents a backprojection matrix, $X^{(k)}$ represents an image estimated at the kth iteration, $\alpha$ represents the step size ($\alpha > 0$). The recursive representation can be rewritten into ta nonrecursive representation as given by:

$$X^{(k)} = \alpha \left[ \sum_{n=0}^{k-1} (I - \alpha A^T W A)^n \right] A^T W P \qquad (3)$$

$$= [I - (I - \alpha A^T W A)^k](A^T W A)^{-1} A^T W P$$

Assume that $(A^T W A)^{-1} = A^{-1} W^{-1} (A^T)^{-1}$ exists. In this case, equation (3) is simplified as:

$$X^{(k)} = [I - (I - \alpha A^T W A)^k] A^{-1} P \qquad (4)$$

Equation (4) corresponds to a noise weighted FBP algorithm, and its filter function in the frequency domain is given by:

$$H(\omega, view) = \left[ 1 - (1 - \alpha \frac{w(view)}{|\omega|})^k \right] |\omega|, \quad H(0, view) = 0 \qquad (5)$$

where $\omega$ represents the frequency variance, w(view) represents the weight value determined for each view, that is, a noise-related weight function at a specific view angle, $\alpha$ represents a parameter which emulates a step size in an iterative algorithm, and k represents a parameter which emulates an iteration number in the iterative algorithm. The view-by-view weighted FBP (to also be referred to as vFBP) algorithm has a shift-invariant point response function (PRF).

In the above embodiment, the reconstruction filter is shaped to emulate a specific iteration result in a predetermined number k of iterations according to the predetermined iterative reconstruction algorithm. The above feature for emulating iterations in the reconstruction filter is not necessarily included in another embodiment for reconstructing an image from the projection data using the view-by-view weighted FBP algorithm.

FIG. 5 is a view showing a scheme of determining a noise weight value for each ray in the image reconstruction method according to this embodiment. In the ray-by-ray weighting scheme, each ray is assigned with a weight value according to a predetermined function such as a maximum or average noise variance in a specific view. FIG. 5 illustrates an X-ray source at a predetermined position and an X-ray detector at a corresponding position. When the X-ray source projects rays, the rays reach the X-ray detector at the corresponding position. The projection data consists of a plurality of rays within a view angle $\theta$ range. For example, a weight value $w(\theta, t_1)$ is determined for a specific ray $t_1$ as a function of the view angle $\theta$ according to the predetermined ray-by-ray weighting scheme. As described above, in one embodiment, determination of a weight value for each ray is performed and completed before shaping a reconstruction filter. In another embodiment, a weight value for each ray is determined as a reconstruction filter.

As the requirement for the shift-invariant PRF is removed, a ray-by-ray weighting scheme is created. By modifying equation (5), a ramp filter with a window in the frequency domain is obtained as:

$$H(\omega, ray) = \left[ 1 - (1 - \alpha \frac{w(ray)}{|\omega|})^k \right] |\omega|, \quad H(0, ray) = 0 \qquad (6)$$

where a weight value, that is, a weighting factor w(ray) is determined based on a ray. The weighting factor w(ray) is determined as a function of the noise variance of the associated ray. k is a parameter indicating a predetermined number of iterations for emulating a specific iteration result according to the predetermined iterative reconstruction algorithm. Assume that h(t, ray) represents the inverse Fourier transform of $H(\omega, ray)$. In this case, h(t, ray) is the spatial-domain kernel of the filter. Let p(r, θ) be the projection at a view θ and ray r and q(r, θ) be the filtered projection. Then, q(r, θ) is defined by:

$$q(r,\theta) = \int_{-\infty}^{\infty} p(t,\theta)h(r-t,r)dt \qquad (7)$$

Since the kernel h(t,r) depends on r, this integral is not convolution. If the filtering procedure is performed in the spatial domain as indicated by equation (7), the calculation cost is the same as that of convolution. The backprojection step in the new rFBP algorithm is the same as that in the conventional FBP algorithm. Therefore, the rFBP algorithm is computationally efficient. On the other hand, the image domain filtering is performed in the 2D Fourier domain with a transfer function, as will be described later in an implementation for the ray-by-ray noise weighted FBP-MAP (rFBP-MAP) algorithm.

A popular approach to assign the weighting factor is to let w(ray) be the reciprocal of the noise variance of ray measurement. This approach is justified by using the likelihood function (that is, the joint probability density function) as the objective function for an optimization problem. If the basic principle that less noisy measurements should be trusted more than noisier measurements is adopted, it is possible to arbitrarily assign a weighting factor as long as a less noisy measurement is assigned with a larger weighting factor w(ray) and a noisier measurement is assigned with a smaller weighting factor w(ray).

The easiest and most efficient way to implement the rFBP algorithm according to the embodiment is to use equation (7) to filter projection in the spatial domain. The present inventors do not currently have an analytical expression for the integration kernel h(t, r). An alternative way to calculate q(r, θ) for each view θ is to calculate equation (7) in the frequency domain as follows.

Step 1: Find the 1 D Fourier transform of p(r, 0) with respect to r, obtaining P(ω, θ).

Step 2.a: For each ray "ray", assign a weighting factor w(ray) and form a frequency domain transfer function as expressed by equation (6). In an implementation, ω represents the frequency index and takes an integer.

Step 2.b: Find the product $Q_{ray}(\omega, \theta) = P(\omega, \theta)H(\omega, ray)$.

Step 2.c: Find the 1D inverse Fourier transform of $Q_{ray}(\omega, \theta)$ with respect to ω, obtaining q(ray, θ).

In the above embodiment, step 1 processes all r's in a row of each view while steps 2.a to 2.c process only one ray at a time. In the view-by-view weighted FBP (vFBP) algorithm, a single weight value or weighting factor w(view) is assigned to all rays in a view. To the contrary, in the ray-by-ray weighted FBP (rFBP) algorithm, a distinct weight value or weighting factor w(ray) is assigned to each ray in a view. As a result, the noise weighting scheme is more accurate in the ray-by-ray weighted FBP (rFBP) algorithm than in the view-by-view weighted FBP (vFBP) algorithm.

As described above with respect to the view-by-view weighted FBP (vFBP) algorithm and the ray-by-ray weighted FBP (rFBP) algorithm, in this embodiment, at least one reconstruction filter is shaped based on parameters including the weight value before generating an image. Furthermore, in the above embodiment, the reconstruction filter is shaped to emulate a specific iteration result in a predetermined number k of iterations according to the predetermined iterative reconstruction algorithm. The above feature for emulating iterations in the reconstruction filter is not necessarily included in another embodiment for reconstructing an image from the projection data using the view-by-view or ray-by-ray weighted FBP algorithm.

After that, a process of generating an image is executed based on the shaped reconstruction filter. In general, noise reduction in image reconstruction is based on the concept that image reconstruction, especially backprojection is addition processing and proper weighting of the projection data substantially reduces the variance of the backprojection.

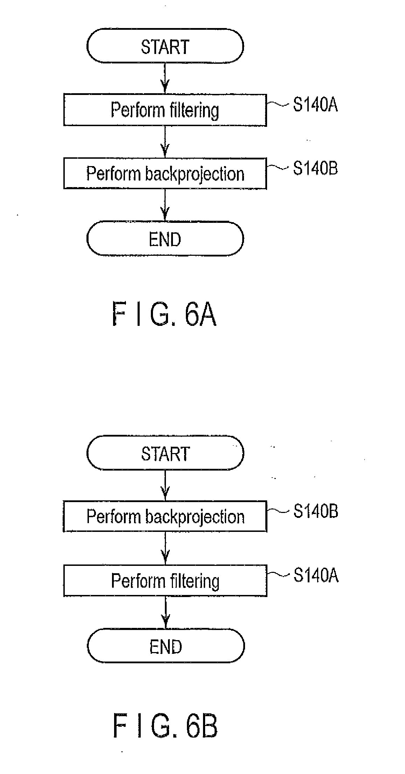

FIG. 6A is a flowchart illustrating the typical procedure of an image reconstruction method including an additional process of generating an image based on a reconstruction filter shaped in advance. Assuming that the FBP algorithm executes tasks on the projection data, the reconstruction filter is applied to the projection data before backprojection is performed. Before step S140A, the reconstruction filter is shaped in advance based on parameters including the noise weight value. The reconstruction filter shaped in step S140A is applied to the projection data. If the projection data undergoes Fourier transform before step S140A, the filtered projection data undergoes inverse Fourier transform before step S140B. In step S140B, the filtered projection data is backprojected. The backprojection generates an image based on the filtered projection data.

FIG. 6B is a flowchart illustrating the typical procedure of another image reconstruction method including an additional process of generating an image based on a reconstruction filter shaped in advance. Assuming that the FBP algorithm executes tasks on the projection data, the reconstruction filter is applied to the projection data after backprojection is performed. If the projection data undergoes Fourier transform before step S140B, the projection data undergoes inverse Fourier transform before step S140B. In step S140B, the projection data is backprojected to generate an image. The shaped reconstruction filter is applied to the backprojected data. Before step S140A, the reconstruction filter is shaped in advance based on parameters including the noise weight value. In step S140A, the shaped reconstruction filter is applied to the projection data.

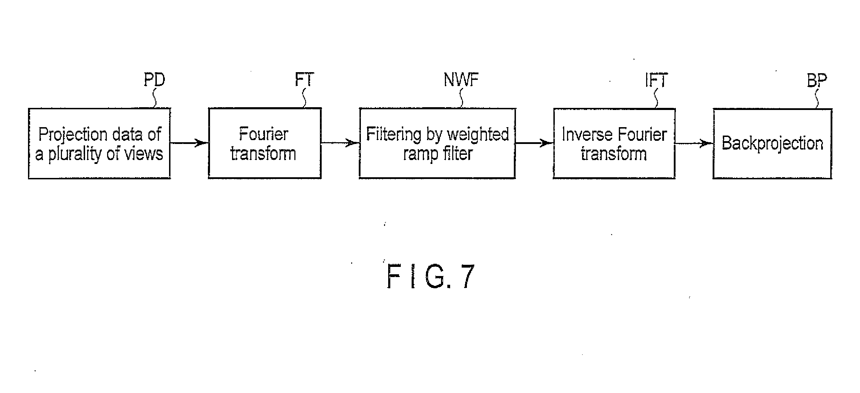

FIG. 7 is a view showing the typical procedure of an image reconstruction method using a noise weighted ramp filter in the FBP algorithm. Assume that in the processing shown in FIG. 7, noise weighted filtering is performed in the Fourier domain before backprojection. As shown in FIG. 7, projection data PD are acquired from a plurality of views. The projection data PD are processed for each view or ray. The projection data PD undergo Fourier transform FT so that the transformed data exist in the Fourier domain or frequency domain. A ramp filter is shaped according to a set of predetermined parameters including a noise weight value. This forms a weighted ramp filter WF. In some embodiments, a plurality of ramp filters are shaped to a plurality of weighted ramp filters WF. These parameters are not limited to a noise weight parameter and may include other parameters. In the exemplary embodiment, the transformed projection data are filtered by a noise weighted ramp filter NWF in the predetermined FBP algorithm. The data filtered in the frequency domain undergo inverse Fourier transform IFT so that the projection data are backprojected in the spatial domain in backprojection BP. Instead of applying the noise weighted ramp filter NWF to the transformed data in the frequency domain, another noise weighted ramp filter NWF2 may be applied to the image. In this case, the noise weighted ramp filter NWF2 to be applied to the image is different from the noise weighted ramp filter NWF to be applied to the transformed data.

The backprojection BP normalizes the above-described varying angle sampling using a weight function while an image is generated. For example, a signal falling within a given angle range including an important structure is sampled at a first angle interval. A signal falling within another angle range outside the region of interest is sampled at a second angle interval larger than the first angle interval. The backprojection BP is an integral over the sampling angle, and angle sampling density compensation is achieved by using a normalization factor, which is essentially a Jacobian factor, in the backprojection integral. A backprojection image is mathematically expressed by:

$$b(x,y) = \int_0^\pi p(t,\theta)\Big|_{t=x\cos\theta+y\sin\theta} d\theta \tag{8}$$

where p represents projection at the angle θ if projection is backprojected for the first time or filtered projection at the angle θ if the FBP algorithm is used, and t represents the location of a detector bin. A discrete representation of equation (8) is given by:

$$b(i,j) = \frac{\pi}{M} \sum_{m=1}^{M} p(n,m)\Big|_{n=INT[x\cos\theta+y\sin\theta]} \tag{9}$$

where n represents a detector location index, m represents a projection angle index, M represents the total number of views where projections are acquired, and "INT" is used to indicate nearest neighbor interpolation. In fact, instead of the "INT" function, linear interpolation between two neighboring detector bins is used.

If simple backprojection as expressed by equation (9) is performed for nonuniform angle sampling, the backprojection depends on a density function d(0) indicating the number of views per unit angle. If, for example, the sampling interval is 1° for 0 < θ < π/4 and 3° for π/4 < 0 < π, the density function is given by:

$$d(\theta) = \begin{Bmatrix} 1 \, for \, 0 \le \theta < \pi/4 \\ 1/3 \, for \, \pi/4 \le \theta < \pi \end{Bmatrix} \tag{10}$$

Equation (9) indicating the backprojection is modified as:

$$b(i,j) = \left( \pi / \sum_{m=1}^{M} \frac{d(1)}{d(m)} \right) \sum_{m=1}^{M} \frac{d(1)}{d(m)} p(n,m)\Big|_{n=INT[x\cos\theta+y\sin\theta]} \tag{11}$$

In this case, the sampling density function is a function of an angle index in instead of the actual angle 9.

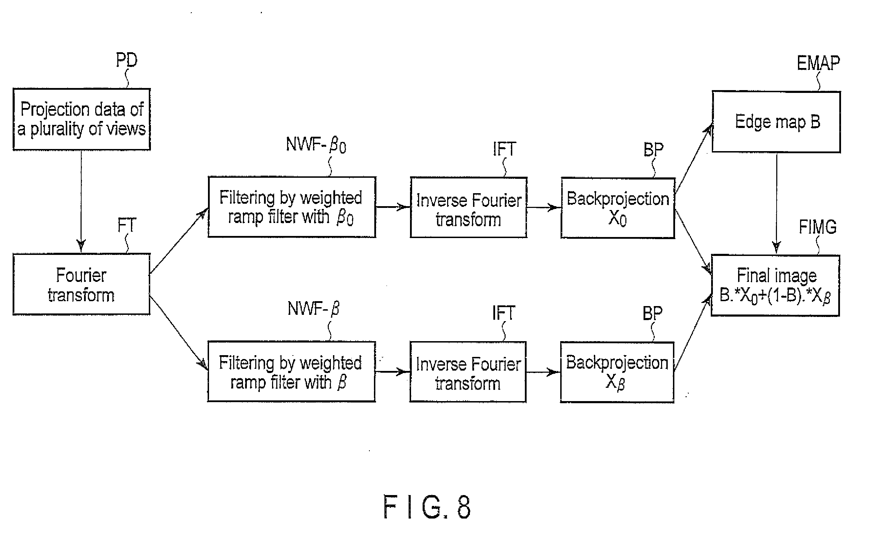

FIG. 8 is a view showing the typical procedure of an image reconstruction method using a pair of noise weighted ramp filters in a predetermined Edge-preserving FBP-MAP algorithm according to the embodiment. In the image reconstruction method shown in FIG. 8, noise weighted filtering is performed in the Fourier domain before backprojecting a pair of projection data to reconstruct images. At last, a final image is generated based on the two images.

As shown in FIG. 8, projection data PD are acquired from a plurality of views. The projection data PD may be processed for each view or ray. The projection data PD undergo Fourier transform FT so that the transformed data are in the Fourier domain or frequency domain. Two ramp filters are separately shaped to weighted ramp filters WF-$\beta_0$ and WF-$\beta$ according to a set of predetermined parameters including a noise weight value. The ramp filters can be additionally shaped by regularization. The filter is a function of a value $\beta$. As the value $\beta$ is larger, the image is smoothed more strongly. If, for example, no smoothing is performed or the image includes no blurring, the value $\beta$ is selected to be zero. The transformed projection data undergo noise weighting in the predetermined FBP algorithm. The noise-weighted projection data are respectively filtered by the regularized ramp filters WF-$\beta_0$ and WF-$\beta$. After that, the filtered projection data undergo inverse Fourier transform IFT in the frequency domain. The projection data having undergone the inverse Fourier transform are backprojected in the spatial domain so as to generate two images $X_0$ and $X_\beta$. The image $X_0$ backprojected without using smoothing or backprojected with minimal smoothing is used to extract edge map information B in an edge map EMAP prepared in advance. A final image FIMG is generated according to a given rule based on the minimally smoothed image $X_0$, the filtered image $X_\beta$, and the extracted edge information.

In the embodiment illustrated in FIG. 8, the edges are preserved during noise reduction using a Bayesian regularization term in an object function given by:

$$F = \left\| P - AX \right\|_W^2 + \beta g(X) \qquad (12)$$

where a penalty function g(X) measures a step in a pixel value relative to its neighbors, A represents a projection matrix, X represents an image array written as a column vector, P represents a projection array written as a column vector, W represents a diagonal matrix consisting of a weight function for each projection, and b represents a relative weighting factor which adjusts the importance of the Bayesian term g(X) with respect to the fidelity term:

$$\left\| P - AX \right\|_W^2$$

which is the first term of equation (12).

The penalty g(X) is a quadratic function of the pixel value step. The pixel value step is calculated as the difference between the value of a pixel of interest and the average value of its neighbors. A large pixel value step corresponds to a large penalty. The penalty function produces a smoothed image.

To suppress the image noise without smoothing out the edges too much, the penalty function g(X) should have different characteristics for edges and non-edge regions. The Huber function having a threshold is used as a penalty function with such ability. If the pixel value step is smaller than the threshold and is classified as noise, the Huber function is quadratic, thereby performing smoothing. If the pixel value step is larger than the threshold and is classified as an edge, the Huber function is linear, thereby performing smaller smoothing.

Another general prior that encourages a piece-wise constant image is the TV (total-variation) measure. For a discrete one-dimensional function g(x), the gradient of the TV measure of g(x) takes only three values, that is, a positive value when g(x) is larger than its left and right neighbors, a negative value when g(x) is smaller than its left and right neighbors, and 0 when g(x) is between its left and right neighbors. In a gradient-type optimization algorithm, a positive gradient value pushes the value of g(x) downward, a negative gradient value pushes the value of g(x) upward, and a gradient value of 0 means that no changes are made. Therefore, the TV prior encourages a monotonic function, suppresses oscillation, and preserves sharp edges.

As illustrated in FIG. 8, the image reconstruction method according to the embodiment applies the Edge-preserving FBP-MAP algorithm to the projection data. $\beta$ is set to $\beta_0$ for one noise weighted ramp filter, that is, zero. Referring to equation (12), $\beta_0 g(x)$ is zero and no smoothing is performed at all. After that, the image $X_0$ is reconstructed without smoothing. On the other hand, for another set of parameters, $\beta$ is set to $\beta$ for another noise weighted ramp filter, that is, a non-zero value. A given amount of smoothing is performed by $\beta$, thereby reconstructing an image $X_\beta$. With this operation, the two images $X_\beta$ and $X_0$ are reconstructed. That is, the first image $X_\beta$ is reconstructed based on rFBP-MAP reconstruction using a non-zero $\beta$ value and the second image $X_0$ is reconstructed based on rFBP reconstruction using a zero $\beta$ value.

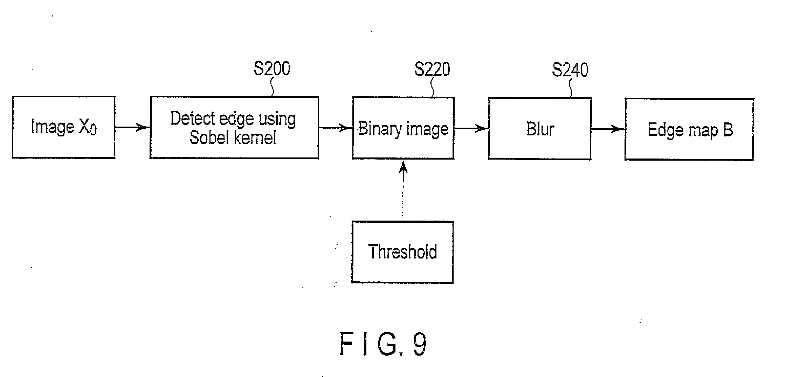

FIG. 9 is a view showing the typical procedure of processing of generating an edge map B shown in FIG. 8. An image $X_0$ is reconstructed by an rFBP reconstruction method using a zero p value. In step S200, a pixel value step is evaluated by edge detection using the Sobel kernel which is essentially the norm of the image gradient. In step S220, an edge map generated by the Sobel kernel is converted into a binary image with a value 0 or 1 according to a predetermined threshold. An image value larger than the threshold is set to 1 and an image value smaller than the threshold is set to 0. In step S240, the binary image is blurred by a predetermined lowpass filter to generate an edge map B. The reason why the binary image is blurred is to smooth the boundary between an edge region and a non-edge region included in the binary image. A final image is generated by a linear combination of $X_b$ and $X_0$ given by:

$$X = B.*X_0 + (1-B).*X_B \qquad (13)$$

where ".*" represents multiplication for each point defined in MATLAB®.

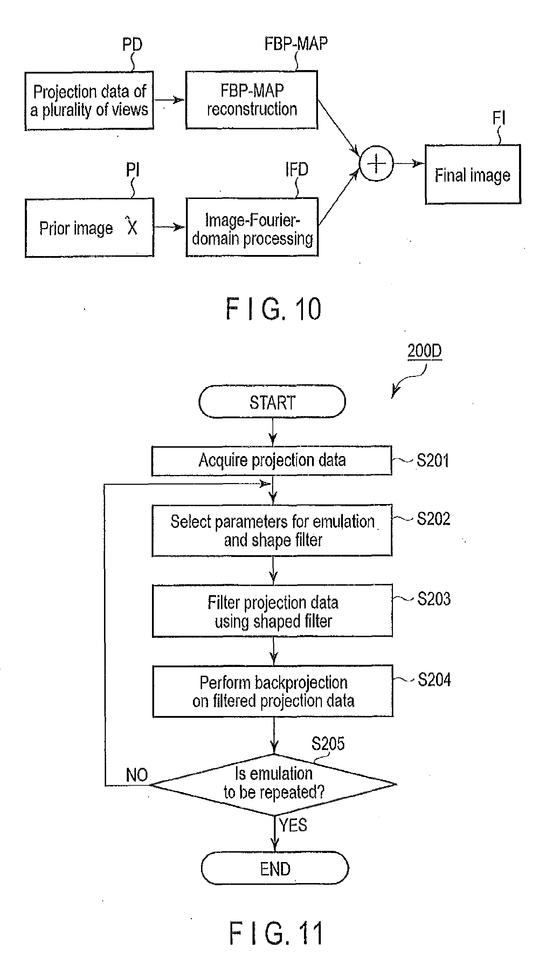

FIG. 10 is a view showing the typical procedure of an image reconstruction method based on an FBP-MAP algorithm using a prior image. In general, to reconstruct an image based on the FBP-MAP algorithm by adding a given prior image

$$\hat{X}$$

the objective function (8) is made more general by:

$$F = \|P - AX\|_W^2 + \beta_1 X^T R_1 X + \beta_2 (X - \hat{X})^T R_2 (X - \hat{X}) \qquad (14)$$

An iterative intermediate solution is expressed by:

$$X^{(k)} = \{I - [I - \alpha(A^T WA + \beta_1 R_1 + \beta_2 R_2)]^k\}(A^T WA + +\beta_1 R_1 + \beta_2 R_2)^{-1}(A^T WP + \beta_2 R_2 \hat{X})$$
$$= \{I - [I - \alpha(A^T WA + \beta_1 R_1 + \beta_2 R_2)]^k\}[I + (A^T WA)^{-1}(\beta_1 R_1 + \beta_2 R_2)]^{-1}[A^{-1}P + \beta_2 (A^T WA)^{-1} R_2 \hat{X}] \qquad (15)$$

An equivalent FBP-MAP algorithm has two Fourier domain filter functions given by:

$$H^{1D}_{k,\beta_1,\beta_2,w}(\omega) = \frac{1 - (1 - \frac{\alpha w}{|\omega|} - \alpha\beta_1 L_1^{1D} - \alpha\beta_2 L_2^{1D})^k}{1 + \frac{|\omega|}{w}(\beta_1 L_1^{1D} + \beta_2 L_2^{1D})}|\omega|, \ \omega \neq 0 \qquad (16)$$

$$G^{2D}_{k,\beta_1,\beta_2,w}(\bar{\omega}) = \frac{1 - (1 - \frac{\alpha w}{|\bar{\omega}|} - \alpha\beta_1 L_1^{2D} - \alpha\beta_2 L_2^{2D})^k}{1 + \frac{|\bar{\omega}|}{w}(\beta_1 L_1^{2D} + \beta_2 L_2^{2D})}\beta_2 \frac{|\bar{\omega}|}{w} L_2^{2D}, \ \bar{\omega} \neq \bar{0} \qquad (17)$$

As shown in FIG. 10, the FBP-MAP algorithm has two inputs including the projection data PD and a prior image PI. A final image FI is an addition image based on a first image derived from the projection data PD and a second image derived from the prior image PI. FBP-MAP reconstruction generates a first reconstruction image based on the detector-Fourier-domain filter as expressed by equation (16) and subsequent backprojection. Image-Fourier-domain processing IFD generates a second image using an image-Fourier-domain filter as expressed by equation (17). The image-Fourier-domain filter may be 2D or 3D.

FIG. 11 is a flowchart illustrating the typical procedure of an image reconstruction method using a noise-weighted iteration-emulation FBP algorithm according to the embodiment. In general, a reconstruction filter emulates a specific iteration result in a predetermined number of iterations. The reconstruction filter is shaped based on parameters including a weight value according to the predetermined iterative reconstruction algorithm. In a noise-weighted iteration-emulation FBP algorithm 200D, in step 201, projection data are acquired. Projection data are acquired in the form of the Radon Transform representing line integrals of an object being imaged. Projection data are acquired in an arbitrary modality such as an X-ray computed tomography apparatus illustrated in FIG. 1, and a PET apparatus and a SPECT device.

In step 202, at least one reconstruction filter is shaped based on a combination of parameters including an iteration number, a noise weight value, and a system matrix. These parameters can be arbitrarily selected. For example, a parameter k is selected to emulate a specific iteration result in a predetermined number of iterations according to the predetermined iterative reconstruction algorithm. In addition, a weight value W determined in advance may be used in conjunction with the iteration parameter k to shape the reconstruction filter to be applied to the projection data. Other filter parameters include a step size in the iterative algorithm and the relative weighting of the Bayesian term so as to accommodate various applications.

After the reconstruction filter is shaped according to the combination of parameters in step S202, the shaped reconstruction filter is applied to the projection data in step 203. The filtered data is backprojected in step 204, thereby generating an image. In step S205, whether to repeat emulation is determined. If emulation is to be repeated, the process advances to step S202 to repeat emulation. If it is determined in step S205 that emulation is not to be repeated, the noise-weighted iteration-emulation FBP algorithm according to this embodiment is terminated.

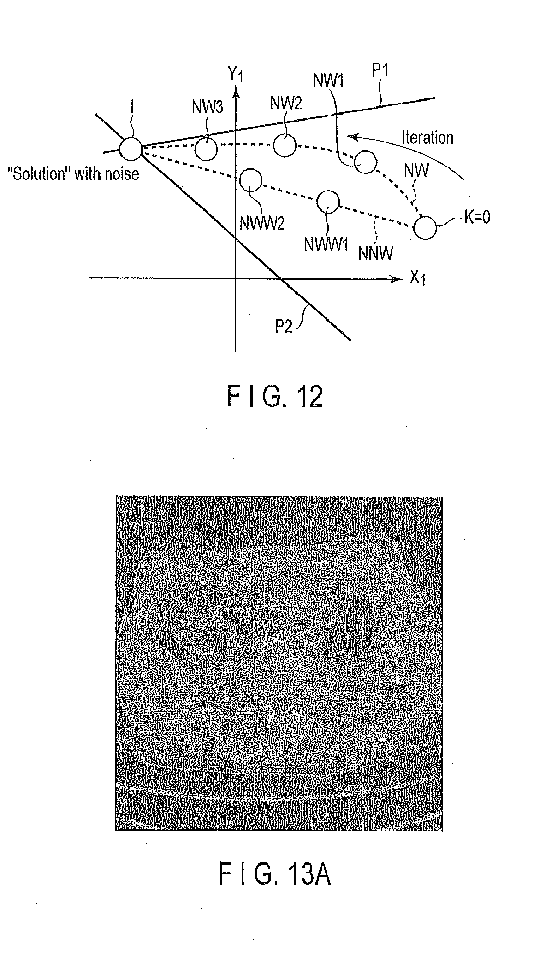

FIG. 12 is a view conceptually showing the effect of the above-described emulated iteration in the noise weighted FBP algorithm according to the embodiment. In conventional CT imaging, noise control is performed using pre-filtering or post-filtering. To the contrary, according to this embodiment, noise is substantially reduced during image reconstruction using the noise weighted and iteration emulated FBP algorithm. Noise reduction in image reconstruction is based on the concept that image reconstruction (especially, backprojection) is addition processing and execution of proper weighting for projections can reduce the variance of the backprojection.

As shown in FIG. 12, two projections or measured data are represented by two projection lines P1 and P2, and a solution or an image I is the intersection of the two projection lines P1 and P2. A horizontal axis $X_1$ and a vertical axis $Y_1$ represent two variables in the image I. If one measurement is made from the image, two image pixels can take values at locations along one straight line. If two independent measurements are made, the values of the two pixels each of which is the intersection of two straight lines are determined. If a third measurement is made and all measurements include noise, three straight lines do not always intersect at one point. In general, when an image consists of many pixels and many inconsistent noisy measurements are made, the solution of an imaging problem may not exist because the measurements may not meet at a single point.

In mathematics, if noisy measurements are inconsistent, they are considered as "not in the range of the projection operator". On the other hand, if measurements are consistent, they are mapped into the range of the projection operator. In fact, the mapping is guaranteed by the backprojection in the FBP algorithm because only data components in the range of the projection operator are backprojected into the image domain. In other words, the projection inconsistency has no contribution to the FBP reconstructed images. Without losing generality, it can be assumed that the measurements are consistent but still noisy and fall within the range of the projection operator even for an image with many pixels, as indicated by a straight line. Unlike an iterative algorithm, the FBP algorithm can provide a unique but noisy solution from a set of noisy projections.

As shown in FIG. 12, to generate a less noisy image, the FBP algorithm is modified to emulate a plurality of iterative steps of the iterative algorithm. A solution trajectory is illustrated from an initial value k = 0 to an ultimate solution k = ∞. A subsequent iteration of the FBP algorithm moves the resultant image with less noise. The iterative algorithm is usually terminated before a true solution is reached. This is particularly true if the true solution is noisy because the measured data contains noise due to a low photon count.

One solution trajectory from k = 0 is a straight line towards the true solution or the image I. The straight line indicates that the emulated iterations are not weighted by a noise variance. Points NNW1 and NNW2 on a straight line indicate intermediate results respectively after a first predetermined number of iterations and a second predetermined number of iterations. In this example, the second predetermined number of iterations is larger than the first predetermined number of iterations, and the intermediate result NNW2 is emulated after the second predetermined number of iterations. On the other hand, intermediate results NW1, NW2, and NW3 are located on a curve, and the curve indicates that the emulated iterations are weighted by the noise variance. The intermediate results NW1, NW2, and NW3 are obtained after a third predetermined number of iterations, a fourth predetermined number of iterations, and a fifth predetermined number of iterations, respectively. In the example, the fifth number is larger than the third and fourth numbers. The third number is smallest. The intermediate result NW3 is emulated after the fifth number of iterations. A solution trajectory is determined based on a set of weighting factors. One weighting factor is assigned to each measurement. A larger factor implies that the associated measurement is more trustworthy. A common approach is to assign a weighting factor that is proportional to the reciprocal of the noise variance of the measurement.

A less noisy pseudo solution in the neighborhood of the true noisy solution is accepted in practice. A procedure for a pseudo solution is parameterized by a predetermined set of parameters including a noise variance weight value and an iteration index k. In general, a pseudo solution with a smaller k corresponds to a smoother image while a pseudo solution with a larger k corresponds to a sharper but noisier image.

Additional embodiments will be described below with respect to a specific combination of the above features according to the embodiment. In one embodiment, ray-by-ray noise weighted FBP using maximum a posteriori (MAP) algorithm, which is designated by rFBP-MAP, is used. Derivation steps for the rFBP-MAP algorithm are very similar to those for the view-by-view weighted FBP-MAP algorithm. Some major derivation steps will be briefly explained below. An objective function is the same as equation (12). If the Bayesian term g(X) is quadratic, the gradient of g(x) is in the form of RX for some matrixes R, and the solution is obtained by the Landweber algorithm represented by:

$$X^{(k+1)} = X^{(k)} + \alpha[A^T W(P - AX^{(k)}) - \beta RX^{(k)}] \qquad (18)$$

The recursive expression by equation (18) has an equivalent non-recursive expression. When the initial condition is zero, the non-recursive expression is given by:

$$X^{(k+1)} = [\sum_{n=0}^{k} (I - \alpha A^T WA - \alpha \beta R)^n] \alpha A^T WP \qquad (19)$$

If $(A^T WA + \beta R)^{-1}$ exists, the total sum in equation (19) has a closed-form given by:

$$X^{(k)} = [I - (I - \alpha A^T WA - \alpha \beta R)^k](A^T WA + \beta R)^{-1} A^T WP \qquad (20)$$

If $((A^T WA)^{-1}$ exists, $(A^T WA + \beta R)^{-1} = [I + \beta((A^T WA)^{-1} R]^{-1}(A^T WA)^{-1}$. If it is assumed that sufficient sampling leads to the existence of Radon inversion $A^{-1}$, it leads to $(A^T WA)^{-1} = A^{-1} W^{-1}(A^T)^{-1}$ and $(A^T WA)^{-1} A^T W = A^{-1}$. Therefore, equation (20) can be simplified as:

$$X^{(k)} = [I - (I - \alpha A^T WA - \alpha \beta R)^k][I + \beta(A^T WA)^{-1} R]^{-1} A^{-1} P \qquad (21)$$

If $A^{-1}$ does not exist for a reason such as an insufficient number of views, it reveals that the FBP algorithm is the pseudo-inverse of the projection operator A and $(A^T WA)^+ A^T W = A^+$, where "+" indicates the pseudo-inverse and $A^+$ is a ramp filtering followed by backprojection $A^T$, Equation (21) still holds with the inverse "-1" by replacing it with the pseudo-inverse "+". Assume, however, that the inverse and the pseudo-inverse are not distinguished. The notation "$A^{-1}P$" is used to represent the conventional FBP reconstruction.

In equation (21), $[I - (I - \alpha.A^T WA - \alpha \beta R)^k]$ represents the windowing effect at the kth iteration of the iterative algorithm, and becomes an identification matrix since k tends to be infinity. $[I + \beta(A^T WA)^{-1} R]^{-1}$ represents the effect of the Bayesian regularization. When $\beta=0$, the Bayesian modification is not effective. Equation (21) provides insights and understanding of how the iterative algorithm processes noise weighting and Bayesian regularization, and can also lead to development of an FBP-type algorithm to execute the same tasks.

Lastly, a Fourier domain expression is derived from the matrix expression so that an FBP-type algorithm is obtained. The matrix $A^{-1}$ is processed as 1 D ramp filtering followed by backprojection or backprojection followed by 2D ramp filtering. When the matrix expression (21) is regarded as a "backprojection first, then filter" algorithm, the image domain filtering in equation (21) is performed in the 2D Fourier domain using a transfer function given by:

$$H_{k,\beta,w}^{2D}(v_x, v_y) = \frac{1 - (1 - \frac{\alpha w}{\|\vec{v}\|} - \alpha \beta L^{2D})^k}{1 + \beta \frac{\|\vec{v}\|}{w} L^{2D}} \|\vec{v}\|, \|\vec{v}\| = \sqrt{v_x^2 + v_y^2} \neq 0 \qquad (22)$$

where $v_x$ and $v_y$ represents frequencies with respect to x and y, respectively.

$$\vec{v} = (v_x, v_y)$$

is a 2D frequency vector.

$$\left\|\vec{v}\right\| = 0$$

As is well known, if the projection operator A is the line-integral (that is, the Radon Transform) in the 2D space and AT is an operator (that is, the backprojection transform), an operator $A^TA$ obtained by combining the projection and backprojection is 2D convolution of the original image with a 2D kernel 1/r. In this case, in the x-y Cartesian coordinate system, we have:

$$r = \sqrt{x^2 + y^2}$$

The 2D Fourier transform of 1/r is given by:

$$1 / \left\|\vec{v}\right\|$$

In equation (22), the Fourier domain equivalence of R is represented by $L^{2D}$.

By using the central slice theorem, image domain 2D filtering is converted into projection domain 1D filtering. A 2D image Fourier domain transfer function (equation (22)) is equivalent to a 1D projection Fourier domain transfer function given by:

$$H_{k,\beta,w}^{1D}(\omega) = \frac{1 - (1 - \dfrac{\alpha w}{|\omega|} - \alpha\beta L^{1D})^k}{1 + \beta \dfrac{|\omega|}{w} L^{1D}} |\omega|, \omega \neq 0 \qquad (23)$$

where $\omega$ represents the frequency for a variable along the detector. When $\omega = 0$, we define:

$$H_{k,\beta,w}^{1D}(0) = 0$$

In equation (23), $L^{1D}$ represents the central slice of $L^{2D}$ of equation (22). In the rFBP-MAP algorithm,

$$H_{k,\beta,w}^{1D}(\omega)$$

is a modified ramp filter expressed in the 1 D Fourier domain. The iteration effect is characterized by:

$$(1 - \frac{\alpha w}{|\omega|} - \alpha\beta L^{1D})^k$$

The Bayesian effect is characterized by:

$$\beta \frac{|\omega|}{w} L^{1D}$$

The ultimate (k = ∞) solution does not depend on the noise weighting w if p = 0 but depends on the noise weighting w if β ≠ 0. It is not necessary to modify backprojection for the rFBP-MAP algorithm.

The noise-dependent weighting factor w in equations (22) and (23) is not a constant. For the view-by-view noise weighting, w is a function of the view angle, that is, w = w(view). For the ray-by-ray noise weighting, w is a function of the ray, that is, w = w(ray). A popular approach to assign the weighting factor is to let w(ray) be proportional to the reciprocal of the noise variance of the ray measurement. This approach is justified by using the likelihood function (that is, the joint probability density function) as the objective function for an optimization problem. The basic principle is that less noisy measurements should be trusted more than noisier measurements. When k tends to be infinity and β tends to be zero, the modified ramp filter (equation (23)) becomes a conventional ramp filter and the rFBP-MAP algorithm reduces to the conventional FBP algorithm.

To perform the rFBP-MAP algorithm, the inverse Fourier transform of

$$H^{1D}_{k,\beta,w}(\omega)$$

is assumed to be

$$h^{1D}_{k,\beta,w}(t)$$

which is the spatial-domain kernel of the 1D modified ramp filter. In the rFBP-MAP algorithm, since w is a function of the ray, w = w(t, θ). Let p(t, θ) be the projection at a view θ and location t on the detector and q(t, θ) be the filtered projection. Then, since the kernel

$$h^{1D}_{k,\beta,w(t,\theta)}(\tau)$$

depends on θ and t, q(t, θ) is defined by:

$$q(t,\theta) = \int_{-\infty}^{\infty} p(\tau,\theta) h^{1D}_{k,\beta,w(t,\theta)}(t-\tau)d\tau \qquad (24)$$

which is not convolution.

If the filtering processing is performed in the spatial domain as in equation (24), the calculation cost is the same as that of convolution. Therefore, the rFBP-MAP algorithm is computationally efficient. The easiest and most efficient way to execute the rFBP-MAP algorithm is to use equation (24) to filter the projections in the spatial domain.

Currently, there is no analytical expression for the integration kernel

$$h^{1D}_{k,\beta,w}(t)$$

An alternative method of calculating q(t, θ) is to calculate equation (24) at each view angle θ in the frequency domain, as follows.

Step 1: Find the 1D Fourier transform of p(t, θ) with respect to t, obtaining P(ω, θ).

Step 2: For each ray t, assign a weighting factor w(t) and form a frequency domain transfer function (equation (23)). ω represents a discrete frequency index and is an integer value of 0, 1, 2,...

Step 3: Find a product.

$$Q_t(\omega,\theta) = P(\omega,\theta)\, H^{1D}_{k,\beta,w(t,\theta)}(\omega)$$

Step 4: Find the 1 D inverse Fourier transform of $Q_1(\omega, \theta)$ with respect to $\omega$, obtaining $q(t, \theta)$.

Step 1 processes all t's in each view while steps 2 to 4 process only one t at a time.

The above-described rFBP-MAP algorithm is not limited to a parallel-beam imaging geometry. The rFBP-MAP algorithm can be extended to other imaging geometries including fan-beam, cone-beam, planar-integrals, and attenuated measurements as long as an FBP algorithm exists. The only modification to the FBP algorithm is in projection data filtering. Each measurement is assigned with a weighting factor, and a frequency-domain filter transfer function is formed as equation (23) independent of an imaging geometry.

To illustrate the effects of the rFBP-MAP algorithm, the torso of a cadaver was scanned using an X-ray computed tomography apparatus with a low dose of X-rays. Images were reconstructed using the FBP algorithm and the rFBP-MAP algorithm. The imaging geometry was cone-beam and the X-ray source trajectory was a circle with a radius of 600 mm. The X-ray detector had 64 rows each having a height of 0.5 mm and 896 channels, and the fan angle was 49.2°. The tube voltage was 120 kV and the current was 60 mA. 1200 views were uniformly sampled over 360°.

Using the Feldkamp algorithm as an FBP algorithm, the data were first weighted with a cosine function, and a 1 D ramp filter was applied to each row of the cone-beam projections. Lastly, cone-beam backprojection was used to generate 3D volume data. To perform the rFBP-MAP algorithm, the 1 D ramp filter was replaced by the ramp filter as expressed by equation (23). The iteration index k was selected as 20,000, and the step size $\alpha$ was set to 2.0. For edge-preserving MAP reconstruction, two $\beta$ values, that is, $\beta$ = 0.0005 and $\beta$ = 0 were used. The Bayesian penalty function g(X) was the common quadratic function of the difference between the central pixel value of a transverse slice and the average value of its four neighbors. In other words, R was a simply Laplacian whose 1 D version was a second order derivative with a kernel of [-1 2 -1]. The noise weighting function was defined by $w(t, \theta) = \exp(-p(t, \theta))$. This embodiment assumed that the transmission measurement approximately had a Poisson distribution, and the line-integral $p(t, \theta)$ was the logarithm of the transmission measurement.

The edge map was obtained for each slice using the 2D Sobel kernel. The edge detection threshold was set to 70% of a maximum pixel value. A 3x3 smooth kernel was applied to a binary image. Application of the smooth kernel blurred edges included in the binary image, and generated the edge map B indicated by equation (13) from the binary image.

The image volume data was reconstructed in a 512×512×512 3D array, and a non-central axial slice is used for display. No other pre-filtering or post-filtering was applied to the reconstruction.

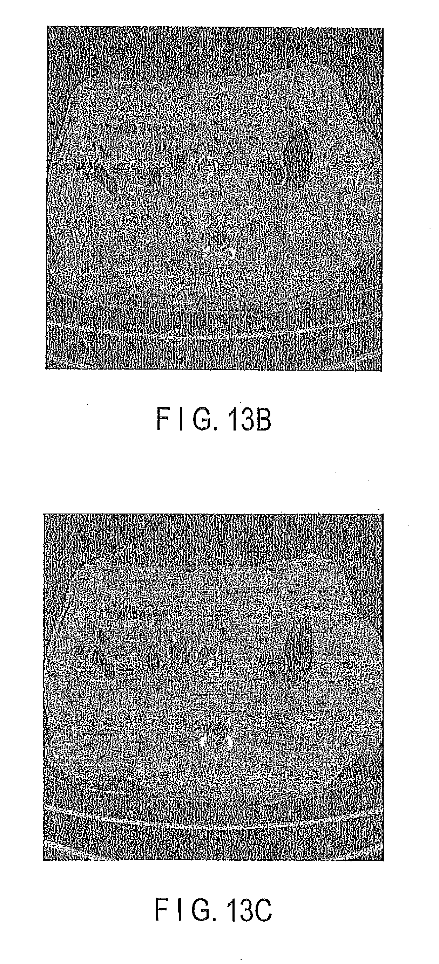

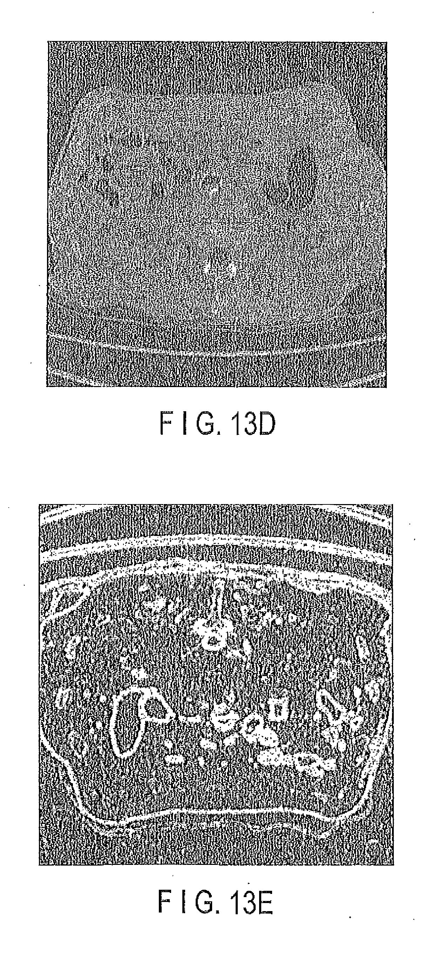

FIG. 13A is a view showing a transverse slice image of the torso region of a cadaver, which is reconstructed based on the conventional FBP Feldkamp algorithm. Due to a low dose, severe streaking artifacts run from left to right across the torso. FIG. 13B is a view showing a transverse slice image of the torso region of the cadaver, which is reconstructed based on rFBP-reconstruction as rFBP-MAP reconstruction with $\beta$ = 0. The ray-by-ray noise weighting in the rFBP algorithm effectively removed the streaking artifacts that appeared in FIG. 13A. FIG. 13C is a view showing a transverse slice image of the torso region of the cadaver, which is reconstructed based on rFBP-MAP reconstruction with $\beta$ = 0.0005. As shown in FIG. 13C, the noise weighting removed the streaking artifacts. Because of a blurring prior, the image looks over-smoothed especially in the edge regions. FIG. 13D is a view showing a transverse slice image of the torso region of the cadaver, which is reconstructed based on rFBP-MAP reconstruction using an edge-preserving prior that is essentially the weighted addition of FIGS. 13B and 13C. The weighted addition image B was extracted from $X_0$ using an edge detection Sobel kernel. The edge map was a binary image. A lowpass filter was used to smooth the edge map so that the transition from an edge region to a non-edge region was smooth. The image shown in FIG. 13D is less noisy than that shown in FIG. 13B but retains the main edges in FIG. 13B unsmoothed. FIG. 13E is a view showing the edge map B used for edge-preserving FBP-MAP reconstruction.

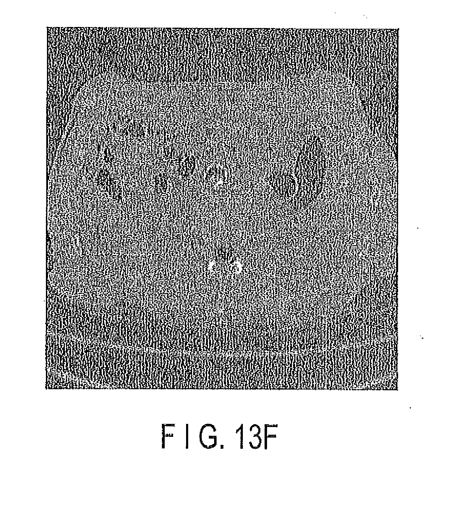

To improve the computational efficiency, the view-by-view noise weighted vFBP-MAP algorithm can be used in many applications. The noise weighting is determined by the largest noise variance at each view. FIG. 13F is a view showing a transverse slice image of the torso region of the cadaver, which is reconstructed based on vFBP-MAP reconstruction. In the vFBP-MAP reconstruction, all parameters were set to be equal to those in the reconstruction shown in FIG. 13D except for the noise weighting function w.

In the image reconstruction according to the embodiment, a noise model is defined by Poisson statistics. Alternatively, a noise model is defined by the compound Poisson statistics of photon intensity measurements with Gaussian distributed electronic noise. In the probability theory, a compound Poisson distribution is the probability distribution of the sum of independent uniformly-distributed random variables, where the number of variables follows the Poisson distribution. A Poisson distribution is well suited to describe the number of incident photons. The number N of photons hitting the detector during the integration time falls within the range from N to Poisson($\lambda$), where $\lambda$ represents the count rate. Each detected photon has random energy $X_i$ whose distribution is described by the X-ray tube polychromatic spectra and detector efficiency. For an energy integration detector, integral energy is given by $Y = \Sigma_N X_i$. Integral energy Y follows the compound Poisson statistics (mean value and variance) given by:

$$E[Y] = E[N] \, E[X]  \tag{25}$$

$$\mathrm{Var}[Y] = E[N] \, E[X^2] = E[N](E[X]^2 + \mathrm{Var}[X])  \tag{26}$$

The concept of a variance gain is represented by W defined by:

$$W = \mathrm{Var}[Y] / E[Y].  \tag{27}$$

Note that for a random variable with a Poisson variance, W = 1. For the compound Poisson variable, we have:

$$W = E[X] + \mathrm{Var}[X] / E[X]  \tag{28}$$

Therefore, the noise variance gain is proportional to the mean effective energy of the X-ray spectra incident on the X-ray detector. In general, several factors as presented below affect the mean spectrum energy.

1) Tube voltage (kVp), which normally varies from 80 to 140 kVp, and determines the upper limit of spectrum energy.
2) Bowtie and spectrum filters and heel effect, which are always present in CT data.
Due to the bowtie filter and heel effect, spectra are different for each pixel. It is thus necessary to independently perform calculation for each detector pixel.
3) Object size and structure. It is well known that objects harden the beam, that is, increase E[X]. The beam hardening effect depends on the path length and attenuation.

The effect of mean spectrum energy on the noise variance gain is used for a compound Poisson model. Acquired data are given by:

$$Z = gY + e  \tag{29}$$

where g represents a detector gain factor and e represents electronic noise with a variance $V_e$. Data are acquired by switching off the X-ray tube, thereby measuring the variance $V_e$. The mean and variance are independently measured in the time direction for each detector pixel. The statistics of the acquired data are given by:

$$E[Z] = g \, E[Y], \qquad \mathrm{Var}[Z] = g^2 \, \mathrm{Var}[Y] + V_e  \tag{30}$$

That is, we have:

$$gW = (\mathrm{Var}[Z] - V_e) / E[Z]  \tag{31}$$

After acquiring projection data for an object, a mean E[Z] and a variance Var[Z] can be measured to calculate gW. Note that a gain g cannot be readily obtained from the projection data. To compensate the effect of an unknown scale g, the projection data are calibrated by air scan data ($W_{OBJ} = W/W_{AIR}$). The compound Poisson model of the noise variance is rewritten as:

$$\mathrm{Var}[Z] = V_e + g \, W \, E[Z]  \tag{32}$$

where W is given by equation (28).
It is to be understood, however, that even though numerous characteristics and advantages of the present invention have been set forth in the foregoing description, together with details of the structure and function of the invention, the disclosure is illustrative only, and that although changes may be made in detail, especially in matters of shape, size, and arrangement of parts, as well as implementation in software, hardware, or a combination thereof, the changes are included in the principles of the invention to the full extent indicated by the broad general meaning of the terms in which the appended claims are expressed.

Some embodiments of the present invention have been described above. However, these embodiments are presented merely as examples and are not intended to restrict the scope of the invention. These embodiments can be carried out in various other forms, and various omissions, replacements, and alterations can be made without departing from the spirit of the invention. The embodiments and their modifications are also incorporated in the scope and spirit of the invention as well as in the invention described in the claims and their equivalents.

**Claims**

1. An X-ray computed tomography apparatus comprising:

   a determining unit configured to determine a weight value for performing weighting on projection data according to at least a predetermined noise model;
   a shaping unit configured to shape at least one reconstruction filter based on parameters including the weight value; and
   a generation unit configured to generate an image based on the reconstruction filter.

2. The X-ray computed tomography apparatus according to claim 1, wherein the determining unit determines the weight value based on the projection data for each view.

3. The X-ray computed tomography apparatus according to claim 2, wherein
   the reconstruction filter is defined by H(w, view) given by:

$$H(\omega, view) = [1 - (1 - \alpha \frac{w(view)}{|\omega|})^k]|\omega|, \quad H(0, view) = 0$$

   where $\omega$ represents a frequency variance, w(view) represents a weight value determined for each view, $\alpha$ represents a parameter which emulates a step size in an iterative algorithm, and k represents a parameter which emulates an iteration number in the iterative algorithm.

4. The X-ray computed tomography apparatus according to claim 1, wherein the determining unit determines the weight value based on the projection data for each ray.

5. The X-ray computed tomography apparatus according to claim 4, wherein
   the reconstruction filter is defined by H(w, ray) given by:

$$H(\omega, ray) = [1 - (1 - \alpha \frac{w(ray)}{|\omega|})^k]|\omega|, \quad H(0, ray) = 0$$

   where $\omega$ represents a frequency variance, w(ray) represents a weight value determined for each ray, $\alpha$ represents a parameter which emulates a step size in an iterative algorithm, and k represents a parameter which emulates an iteration number in the iterative algorithm.

6. The X-ray computed tomography apparatus according to claim 1, wherein the generation unit applies the reconstruction filter to the projection data before execution of backprojection.

7. The X-ray computed tomography apparatus according to claim 1, wherein the generation unit applies the reconstruction filter to the projection data after execution of backprojection.

8. The X-ray computed tomography apparatus according to claim 1, wherein the noise model includes Poisson statistics.

9. The X-ray computed tomography apparatus according to claim 1, wherein the noise model is described by compound Poisson statistics of photon intensity measurements containing electronic noise with a Gaussian distribution.

10. The X-ray computed tomography apparatus according to claim 1, further comprising a regularization unit configured to perform regularization to shape the reconstruction filter.

11. The X-ray computed tomography apparatus according to claim 10, wherein the image is further generated based on a reference image.

12. The X-ray computed tomography apparatus according to claim 10, wherein
the shaping unit shapes a first reconstruction filter and a second reconstruction filter as the reconstruction filters,
the first reconstruction filter is shaped to generate a first image for preserving an edge, and the second reconstruction filter is shaped to generate a second image for generating an edge map by reducing noise, and
the image is generated based on the first image, the second image, and the edge map.

13. The X-ray computed tomography apparatus according to claim 1, wherein the shaping unit shapes the reconstruction filter to emulate a specific iteration result in a predetermined number of iterations according to a predetermined iterative reconstruction algorithm.

14. The X-ray computed tomography apparatus according to claim 1, wherein the shaping unit shapes the reconstruction filter based on a system matrix.

15. The X-ray computed tomography apparatus according to claim 14, wherein the system matrix designates nonuniform sampling of the projection data.

16. An image reconstruction method comprising:

determining a weight value for performing weighting on projection data according to at least a predetermined noise model;
shaping at least one reconstruction filter based on parameters including the weight value; and
generating an image based on the reconstruction filter.

F I G. 1

```
┌─────────────────────────────────────────────────┐
│   ┌─────────────────────────────────┐            │
│   │  Noise weight determining unit  │─ 114A   ─ 114
│   └─────────────────────────────────┘            │
│                    │                             │
│   ┌─────────────────────────────────┐            │
│   │       Filter shaping unit       │─ 114B      │
│   └─────────────────────────────────┘            │
│                    │                             │
│   ┌─────────────────────────────────┐            │
│   │      Image generation unit      │─ 114C      │
│   └─────────────────────────────────┘            │
└─────────────────────────────────────────────────┘
```

F I G. 2

```
        ┌─────────────────┐
        │      START      │
        └─────────────────┘
                 │
        ┌─────────────────────┐
        │ Determine weight value │─ S100
        └─────────────────────┘
                 │
        ┌─────────────────┐
        │   Shape filter  │─ S120
        └─────────────────┘
                 │
        ┌─────────────────┐
        │  Generate image │─ S140
        └─────────────────┘
                 │
        ┌─────────────────┐
        │       END       │
        └─────────────────┘
```

F I G. 3

X ray tube ⃝A

$w(\theta_1)$

C'

B'

$w(\theta_2)$

⃝B

A'

$w(\theta_3)$

X ray detector

C

# F I G. 4

$w(\theta,t_1)$

$w(\theta,t_2)$

X ray tube ⃝

$w(\theta,t_3)$

X ray detector

$w(\theta,t_4)$

$w(\theta,t_5)$

# F I G. 5

EP 2 881 039 A1

START

Perform filtering ⌐~ S140A

Perform backprojection ⌐~ S140B

END

F I G. 6A

START

Perform backprojection ⌐~ S140B

Perform filtering ⌐~ S140A

END

F I G. 6B

21

PD

Projection data of
a plurality of views

FT

Fourier
transform

NWF

Filtering by weighted
ramp filter

IFT

Inverse Fourier
transform

BP

Backprojection

F I G. 7

EP 2 881 039 A1

PD

Projection data of
a plurality of views

FT

Fourier
transform

NWF-$\beta_0$

Filtering by weighted
ramp filter with $\beta_0$

IFT

Inverse Fourier
transform

BP

Backprojection
$X_0$

EMAP

Edge map B

FIMG

Final image
$B.*X_0+(1-B).*X_\beta$

NWF-$\beta$

Filtering by weighted
ramp filter with $\beta$

IFT

Inverse Fourier
transform

BP

Backprojection
$X_\beta$

F I G. 8

Image X₀ → Detect edge using Sobel kernel (S200) → Binary image (S220) → Blur (S240) → Edge map B

Threshold → Binary image

F I G. 9

EP 2 881 039 A1

PD                          FBP-MAP

```
┌─────────────────┐      ┌─────────────────┐
│ Projection data of│ ──→ │    FBP-MAP      │
│ a plurality of views│    │  reconstruction │
└─────────────────┘      └─────────────────┘
```
                                                            FI
                                              ⊕ ──→ ┌──────────────┐
PI                          IFD                     │  Final image  │
```
┌─────────────────┐      ┌─────────────────┐       └──────────────┘
│  Prior image  X̂  │ ──→ │ Image-Fourier-  │
│                 │      │ domain processing│
└─────────────────┘      └─────────────────┘
```

F I G. 10

200D

```
        ┌─────────────┐
        │    START    │
        └─────────────┘
               │
        ┌─────────────────────┐
        │ Acquire projection data │ ~ S201
        └─────────────────────┘
               │
        ┌─────────────────────┐
        │ Select parameters for emulation │ ~ S202
        │      and shape filter   │
        └─────────────────────┘
               │
        ┌─────────────────────┐
        │  Filter projection data │ ~ S203
        │   using shaped filter   │
        └─────────────────────┘
               │
        ┌─────────────────────┐
        │  Perform backprojection │ ~ S204
        │ on filtered projection data│
        └─────────────────────┘
               │        S205
    NO      ◇ Is emulation
   ┌────────  to be repeated? ◇
   │           │
   │          YES
   │        ┌─────────────┐
   │        │     END     │
   │        └─────────────┘
```

F I G. 11

F I G. 12

F I G. 13A

FIG. 13B

FIG. 13C

F I G. 13D

F I G. 13E

F I G. 13F

## INTERNATIONAL SEARCH REPORT

| International application No. |
| --- |
| PCT/JP2013/070659 |

### A. CLASSIFICATION OF SUBJECT MATTER
*A61B6/03*(2006.01)i

According to International Patent Classification (IPC) or to both national classification and IPC

### B. FIELDS SEARCHED

Minimum documentation searched (classification system followed by classification symbols)
A61B6/03

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X<br>A | Akifumi FUJITA, "Evaluation of Ultra-low-dose Thoracic Multi-detector-row CT using Different Reconstruction Kernels and New Reconstruction Algorithms", Japan Radiological Society Zasshi, 25 November 2003 (25.11.2003), vol.63, no.9, pages 588 to 590 | 1,6,16<br>2-5,7-15 |
| A | JP 2012-70814 A (GE Medical Systems Global Technology Co., L.L.C.), 12 April 2012 (12.04.2012), entire text; all drawings (Family: none) | 1-16 |

☐ Further documents are listed in the continuation of Box C.        ☐ See patent family annex.

| | | |
| --- | --- | --- |
| * | Special categories of cited documents: | |
| "A" | document defining the general state of the art which is not considered   to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 29 October, 2013 (29.10.13) | 05 November, 2013 (05.11.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)